(19) Europäisches Patentamt
European Patent Office
Office européen des brevets

(11) Numéro de publication: **0 078 562**
**B1**

(12) # FASCICULE DE BREVET EUROPEEN

(45) Date de publication du fascicule du brevet: **29.01.86**

(21) Numéro de dépôt: **82201292.8**

(22) Date de dépôt: **18.10.82**

(51) Int. Cl.⁴: **A 01 N 37/02,** A 01 N 37/10, A 01 N 37/12, A 01 N 37/40, A 01 N 43/08, C 07 C 153/07, C 07 D 307/68

(54) **Compositions acaricides.**

(30) Priorité: **29.10.81 LU 83725**

(43) Date de publication de la demande:
**11.05.83 Bulletin 83/19**

(45) Mention de la délivrance du brevet:
**29.01.86 Bulletin 86/05**

(84) Etats contractants désignés:
**AT BE CH DE FR GB IT LI NL SE**

(56) Documents cités:
**FR-A-1 288 659**
**GB-A-1 594 962**
**NL-A-6 914 354**
**NL-A-7 515 243**
**US-A-2 259 869**
**US-A-4 095 970**
**JOURNAL OF THE SCIENCE OF FOOD AND AGRICULTURE, vol. 8, 1957, pages 561-565, Chemical Industry, Londres (GB); R.F. BROOKES et al.: "The toxicity of organic sulphides to the eggs and larvae of the glasshouse red spider mite. II. Miscellaneous sulphides"**

(73) Titulaire: **SOCIETE D'ETUDES ET DE REALISATIONS SCIENTIFIQUES, en abrégé S.E.R.E.S.C.I., S.P.R.L.**
**Avenue Jean Jaurès, 46**
**B-1030 Bruxelles (BE)**

(72) Inventeur: **De Muylder, Jean Marie deceased (BE)**

(74) Mandataire: **De Brabanter, Maurice et al**
**Bureau VANDER HAEGHEN 63 Avenue de la Toison d'Or**
**B-1060 Bruxelles (BE)**

**EP 0 078 562 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Courier Press, Leamington Spa, England.

**Description**

La présente invention est relative à des compositions acaricides contenant, comme ingrédient actif, un composé organique sulfuré.

L'invention concerne, en particulier, des compositions acaricides agissant sur les sarcoptes, psoriotes, chiorptes, tiques, tétraniques et autres acares, sous forme d'oeufs, de larves et d'acares adultes. Ces compositions acaricides peuvent être utilisées dans le domaine vétérinaire, par exemple pour le traitement de la gale, ainsi que dans le domaine phytopharmaceutique, par exemple pour lutter contre les araignées, telles que les tétraniques.

On sait, par le brevet belge n° 837.074, que plusieurs composés organiques sulfurés, tels que le thiobenzoate de S-octyle, le thiobenzoate de S-phényle, le thiobenzoate de S-benzyle et le thioacétate de S-chlorophényle possèdent des propriétés acaricides. Parmi ces composés acaricides connus, le thiobenzoate de S-octyle possède de ramarquables propriétés acaricides.

On a constaté à présent qu'un grand nombre de composés organiques sulfurés ont des propriétés acaricides sensiblement meilleures que le thiobenzoate de S-octyle.

L'invention concerne, dès lors, une composition acaricide contenant comme ingrédient actif, au moins un composé organique sulfuré répondant à la formule I suivante:

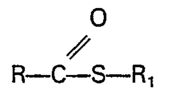

(I)

dans laquelle R et $R_1$ sont des radicaux organiques différents, caractérisée en ce que R désigne un radical choisi parmi les radicaux méthyle, éthyle, propyle, butyle, hexyle et heptyle à chaîne droite ou ramifiée, le radical phényle éventuellement méthoxylé et le radical furyle-2 et $R_1$ désigne un radical chosi parmi les radicaux octyle, dodécyle et hexadécyle ou un groupe thioacyl-hexaméthylène de formule II suivante:

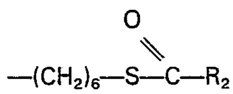

(II)

dans laquelle $R_2$ est identique à R, étant entendu que R ne représente pas un radical phényle, lorsque $R_1$ représente un radical octyle.

Plus particulièrement, l'invention a également trait à des compositions acaricides contenant, comme ingrédient actif, un thioester du type di(acylmercapto)-1,6-hexane répondant à la formule III suivante:

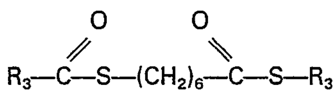

(III)

dans laquelle les symboles $R_3$ désignent des radicaux identiques choisis parmi les radicaux phényle et furyle-2.

Une liste des composés de formule I ou III qui peuvent être contenus dans les compositions acaricides suivant l'invention est donnée dans les tableaux 1 et 2 suivants:

TABLEAU 1

| Numéro de code | R | $R_1$ |
|---|---|---|
| 272 | $CH_3$ | $-C_8H_{17}$ |
| 273 | $CH_3$ | $-C_{12}H_{25}$ |
| 274 | $CH_3$ | $-C_{16}H_{33}$ |
| 282 | $n-C_3H_7$ | $-C_8H_{17}$ |
| 283 | $n-C_3H_7$ | $-C_{12}H_{25}$ |
| 284 | $n-C_3H_7$ | $-C_{16}H_{33}$ |
| 292 | $iso-C_3H_7$ | $-C_8H_{17}$ |
| 293 | $iso-C_3H_7$ | $-C_{12}H_{25}$ |
| 294 | $iso-C_3H_7$ | $-C_{16}H_{33}$ |
| 302 | $(CH_3)_3C-$ | $-C_8H_{18}$ |
| 303 | $(CH_3)_3C-$ | $-C_{12}H_{25}$ |
| 304 | $(CH_3)_3C-$ | $-C_{16}H_{33}$ |
| 312 | $n-C_7H_{15}$ | $-C_8H_{17}$ |
| 313 | $n-C_7H_{15}$ | $-C_{12}H_{25}$ |
| 314 | $n-C_7H_{15}$ | $-C_{16}H_{33}$ |
| 242 | $-C_6H_5$ | $-C_{12}H_{25}$ |
| 261 | $p-CH_3O-C_6H_5-$ | $-C_8H_{17}$ |
| 263 | $p-CH_3O-C_6H_5-$ | $-C_{12}H_{25}$ |
| 252 | furyle-2 | $-C_8H_{17}$ |
| 254 | furyle-2 | $-C_{12}H_{25}$ |

TABLEAU 2

| Numéro de code | Formule II |
|---|---|
| 275 | 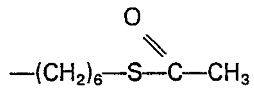 |
| 285 | 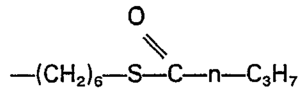 |
| 295 | 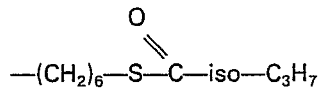 |
| 305 | 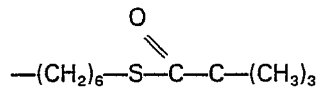 |
| 315 | 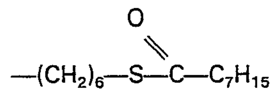 |
| 243 | 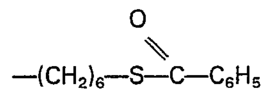 |
| 264 | 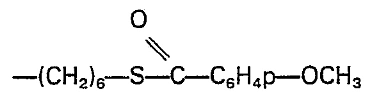 |
| 253 | 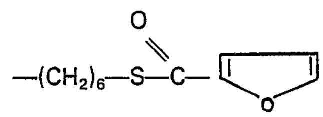 (furyle-2) |

Les compositions acaricides suivant la présente invention peuvent être liquides ou solides, par exemple sous forme de liquides à pulvériser, de poudres mouillables, de shampoings, de solutions à frictionner ou d'onguents.

Les composés de formule I peuvent être préparés en faisant réagir un chlorure d'acide de formule IV suivante:

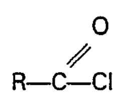 (IV)

dans laquelle R a les signifactions indiquées plus haut, avec un thiol de formule V suivante:

$$R_2—SH \quad (V)$$

dans laquelle $R_2$ a les significations indiquées plus haut.

Les dithioacylmercapto-1,6-hexanes de formule III peuvent être préparés en faisant réagir deux moles d'un chlorure d'acide de formule VI suivante:

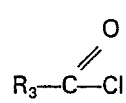 (VI)

dans laquelle $R_3$ a les significations indiquées plus haut, avec une mole d'hexane-dithiol de formule $HS—(CH_2)_6—SH$.

Les exemples 1 à 28 suivants décrivent la préparation de thioesters de formule I et de dithioacylmercapto-1,6-hexanes de formule III utilisables dans les compositions acaricides suivant l'invention.

Exemples 1 à 20

Les thioesters de formule I se préparent comme suit:

Un mélange de quantités équimolaires d'un chlorure d'acide de formule IV et d'un thiol de formule V est chauffé à une température de 130—140°C. Cette température est maintenue jusqu'à ce qu'il ne se dégage plus d'acide chlorhydrique (durée totale de la réaction: 4 heures), selon la réaction suivante:

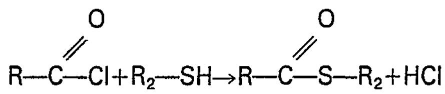

Le résidu est purifié soit par distillation sous pression réduite, soit par recristallisation dans de l'isopropanol.

Le rendement atteint 85 à 95%.

Le tableau 3 suivant donne les constantes physiques, c'est-à-dire les points d'ébullition (E) et les points de fusion (F) en °C, de même que les teneurs calculées et trouvées en soufre des thioesters de formule III qui ont été préparés, les indices des points d'ébullition représentant les pressions en millimètres de mercure auxquelles les points d'ébullition ont été mesurés.

TABLEAU 3

| Exemple | Numéro de code | Nom du thioester | E ou F | Teneur en soufre | |
|---|---|---|---|---|---|
| | | | | Trouvé | Calculé |
| 1 | 272 | Thioacétate de S-octyle | $E_{14}$:122° | 17,1 | 17,09% |
| 2 | 273 | Thioacétate de S-dodécyle | $E_{0,3}$:102° | 13,25 | 13,12 |
| 3 | 274 | Thioacétate de S-hexadécyle | F:28°3—28°6 | 10,4 | 10,67 |
| 4 | 282 | Thiobutyrate de S-octyle | $E_{0,3}$:80° | 14,6 | 14,82 |
| 5 | 283 | Thiobutyrate de S-dodécyle | $E_{0,5}$:130° | 12,1 | 11,8% |
| 6 | 284 | Thiobutyrate de S-hexadécyle | $E_{0,4}$:172° | 10,0 | 9,8 |
| 7 | 292 | Thioisobutyrate de S-octyle | $E_{0,3}$:77° | 14,7 | 14,8 |
| 8 | 293 | Thioisobutyrate de S-dodécyle | $E_{0,3}$:111° | 11,8 | 11,8 |
| 9 | 294 | Thioisobutyrate de S-hexadécyle | $E_{0,3}$:152° | 10,0 | 9,8 |
| 10 | 302 | Thiopivalate de S-octyle | $E_{1}$:108° | 13,9 | 13,9 |
| 11 | 303 | Thiopivalate de S-dodécyle | $E_{0,3}$:124° | 11,4 | 11,2 |
| 12 | 304 | Thiopivalate de S-hexadécyle | $E_{0,35}$:157° | 9,4 | 9,4 |
| 13 | 312 | Thiooctanoate de S-octyle | $E_{0,3}$:128° | 11,5 | 11,8 |
| 14 | 313 | Thiooctanoate de S-dodécyle | $E_{0,3}$:159° | 9,9 | 9,8 |
| 15 | 314 | Thiooctanoate de S-hexadécyle | F:36°4—37° | 8,55 | 8,3 |
| 16 | 242 | Thiobenzoate de S-dodécyle | $E_{0,25}$:167° | 10,2 | 10,5 |
| 17 | 261 | p-Thioanisoate de S-octyle | $E_{0,5}$:167° | 11,1 | 11,4 |
| 18 | 263 | p-Thioanisoate de S-dodécyle | F:31°1 | 9,4 | 9,5 |
| 19 | 252 | Thiofuroate-2 de S-octyle | $E_{0,8}$:135° | 13,2 | 13,3 |
| 20 | 254 | Thiofuroate-2 de S-dodécyle | $E_{0,5}$:165° F:28°7 | 20,5 | 10,8 |

Exemples 21 à 28

Les dithioacylmercapto-1,6-hexanes de formule III se préparent en mélangeant deux moles du chlorure d'acide de formule VI avec une mole d'hexane-dithiol et en chauffant progressivement le mélange jusqu'à une température de 130—140°C. Cette température est maintenue jusqu'à ce qu'il n'y ait plus de dégagement d'acide chlorhydrique. La réaction peut être schématisée comme suit:

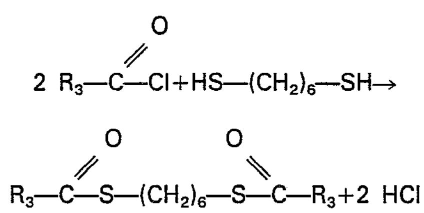

Le produit de la réaction est purifié soit par distillation sous pression réduite, soit par recristallisation dans de l'isopropanol.

Le rendement de la réaction est compris entre 85 et 95%.

Le tableau 4 suivant donne les constantes physiques, c'est-à-dire les points d'ébulliton et de fusion, de même que les teneurs en soufre calculées et trouvées des composés préparés.

TABLEAU 4

| Exemple | Numéro de code | Nom du S-thioester du type di(acylmercapto) -1,6-hexane | E ou F | Teneur en soufre | |
|---|---|---|---|---|---|
| | | | | Trouvé | Calculé |
| 21 | 275 | Diacétylmercaptohexane | $E_{0,4}$:98° | 27,3 | 27,3% |
| 22 | 285 | Dibutyrylmercaptohexane | $E_{0,5}$:139° | 22,2 | 22,1 |
| 23 | 295 | Diisobutyrylmercaptohexane | $E_{0,5}$:136° | 22,1 | 22,1 |
| 24 | 305 | Dipivaloylmercaptohexane | $E_{0,4}$:145° | 20,0 | 20,1 |
| 25 | 315 | Dioctanoylmercaptohexane | F:39—39°1 | 15,7 | 15,9 |
| 26 | 243 | Dibenzoylmercaptohexane | F:51°8—52°4 | 18,2 | 17,9 |
| 27 | 264 | Di-p-anisoylmercaptohexane | F:86°8—87° | 15,5 | 15,3 |
| 28 | 253 | Difuroyl-2-mercaptohexane | F:108°5—109°1 | 18,7 | 18,9 |

Le tableau 5 suivant indique la densité et 1' indice de réfraction de quelques composés utilisés comme ingrédients actifs dans les compositions acaricides suivant la présente invention.

TABLEAU 5

| Numéro de code | Densité $D_4^{22}$ | Indice de réfraction $n_D^{22}$ |
|---|---|---|
| 272 | 0,905 | 1,4615 |
| 273 | 0,8719 | 1,4620 |
| 282 | 0,8588 | 1,4625 |
| 283 | 0,8754 | 1,4632 |
| 284 | 0,8770 | 1,4642 |
| 292 | 0,8842 | 1,4592 |
| 293 | 0,8760 | 1,4621 |
| 294 | 0,8666 | 1,4628 |
| 302 | 0,8803 | 1,4590 |
| 303 | 0,8709 | 1,4618 |
| 304 | 0,8664 | 1,4635 |
| 312 | 0,8772 | 1,4648 |
| 313 | 0,8710 | 1,4656 |
| 242 | 0,9554 | 1,5175 |
| 261 | 1,0245 | 1,5435 |
| 252 | 1,0153 | 1,517 |
| 275 | 1,0496 | 1,5075 |
| 285 | 1,0060 | 1,4970 |
| 295 | 0,9983 | 1,4946 |
| 305 | 0,9820 | 1,4905 |

Les exemples 29 à 33 décrivent quelques compositions acaricides suivant le présente invention.

Exemple 29

| Liquide à pulvériser | % en poids |
|---|---|
| Ingrédient actif de formule I | 50% |
| Solvant (tel que xylène) | 45% |
| Agent mouillant anionique ou non ionique tel que Tensiofix B 7416 (marque déposée-TENSIA, Belgique) | 2,1% |
| Tensiofix B 7435 (marque déposée-TENSIA, Belgique) | 2,9% |

Exemple 30

| Shampoing | % en poids |
|---|---|
| Ingrédient actif de formule I | 25% |
| Texapon (marque déposée—HENKEL AG, R.F.A.) | 8% |
| Eau, q.s. ad | 100% |

Exemple 31

| Poudre mouillable | % en poids |
|---|---|
| Ingrédient actif de formule I | 10% |
| Nekal (marque déposée) | 2,5% |
| Wettol D1 (marque déposée) | 5,0% |
| Kieselguhr | 25,0% |
| Argile Borden | 57,5% |

Exemple 32

| Solution à tamponner ou à frictionner | % en poids |
|---|---|
| Ingrédient actif de formule I ou III | 2,5% |
| Huile de paraffine | 97,5% |

Exemple 33

| Onguent | % en poids |
|---|---|
| Ingrédient actif de formule I ou III | 2,5% |
| Excipient pour onguent cétylique (alcool cétylique, lanoline, paraffine) | 97,5% |

Des composés répondant aux formules I et III ont été soumis à des essais en vue de comparer leur activité acaricide à celle d'un composé connu, en l'occurrence le thiobenzoate de S-octyle (DB 167).

On a élevé des acares du type Tetranychus urticae sur des petits plants de haricots verts (Phaseolus vulgaris) en serre à une température minimale de 25°C. Les acares de première génération ont été prélevés sur une infestation naturelle de vignes de la vallée du Rhône.

Par transfert de fragments de feuilles infestées d'acares sur des plantes hôtes non infestées, deux feuilles parcelle ont chaque fois été infestées pendant une durée de 12 heures. Avant le traitement à l'aide d'une composition acaricide du type décrit dans l'exemple 42 ou 44, le nombre d'adultes et de larves a été compté sur les deux feuilles infestées.

Le traitement à l'aide des compositions contenant un ingrédient actif (composé connu DB 167 ou composé de formule I ou III) a été effectué en pulvérisant environ 20 cc des compositions sur deux feuilles à l'aide d'un atomiseur de parfum. Après le traitement, les plantes ont été placées dans la serre à une température moyenne de 22 à 25°C.

L'activité des composés a été déterminée à des doses de 4000 et 2000 ppm d'ingrédient actif (i.a.), 2, 5 et 10 hours après le traitement en comptant le nombre de formes vivantes de l'acare. Le pourcentage de contrôle (C) a été calculé en utilisant la formule suivante:

$$\% \text{ Contrôle} = 100 \times \left(1 - \frac{Ta \times Cb}{Tb \times Ca}\right)$$

dans laquelle Tb et Ta sont le nombre d'acares respectivement avant et après le traitement dans des parcelles traitées, tandis que Ca et Cb sont le nombre d'acares respectivement avant traitement et après traitement dans des parcelles non traitées.

Le tableau 6 suivant donne les résultats des essais.

TABLEAU 6

| Numéro de code des composés | ppm i.a. | Moyenne de deux essais | | | | | | |
|---|---|---|---|---|---|---|---|---|
| | | Avant traitement | Jours après traitement | | | | | |
| | | | 2 | | 5 | | 10 | |
| | | Nombre d'acares | Nombre d'acares | %C | Nombre d'acares | %C | Nombre d'acares | %C |
| DB 167 (composé connu) | 4000 | 102 | 0 | 100 | 0 | 100 | 20 | 93,1 |
| | 2000 | 97 | 21 | 80,7 | 24 | 85 | 23 | 91,7 |
| DB 272 | 4000 | 82 | 16 | 82,6 | 11 | 91,8 | 15 | 93,6 |
| | 2000 | 123 | 35 | 74,6 | 18 | 91,1 | 32 | 90,9 |
| DB 273 | 4000 | 105 | 8 | 93,2 | 4 | 97,7 | 8 | 97,3 |
| | 2000 | 104 | 23 | 80,2 | 17 | 90,1 | 19 | 90,2 |
| DB 274 | 4000 | 119 | 12 | 91 | 5 | 97,4 | 2 | 99,4 |
| | 2000 | 97 | 14 | 87,1 | 8 | 95 | 4 | 98,6 |
| DB 282 | 4000 | 117 | 11 | 91,6 | 5 | 97,4 | 15 | 95,5 |
| | 2000 | 140 | 45 | 71,3 | 17 | 92,6 | 21 | 94,7 |
| DB 283 | 4000 | 77 | 1 | 98,8 | 0 | 100 | 6 | 97,3 |
| | 2000 | 115 | 10 | 92,2 | 4 | 97,9 | 18 | 94,5 |
| DB 284 | 4000 | 93 | 0 | 100 | 0 | 100 | 0 | 100 |
| | 2000 | 105 | 14 | 88,1 | 6 | 96,5 | 2 | 99,3 |
| DB 292 | 4000 | 107 | 0 | 100 | 0 | 100 | 9 | 97,1 |
| | 2000 | 88 | 5 | 94,9 | 3 | 97,9 | 8 | 96,8 |
| DB 293 | 4000 | 81 | 11 | 87,9 | 8 | 94 | 14 | 93,9 |
| | 2000 | 117 | 27 | 79,4 | 30 | 84,4 | 37 | 88,9 |
| DB 294 | 4000 | 117 | 0 | 100 | 1 | 99,5 | 0 | 100 |
| | 2000 | 128 | 20 | 86 | 10 | 95,2 | 3 | 99,2 |
| DB 302 | 4000 | 100 | 2 | 98,2 | 2 | 98,8 | 70 | 75,5 |
| | 2000 | 120 | 7 | 94,8 | 7 | 96,5 | 65 | 81 |
| DB 303 | 4000 | 92 | 0 | 100 | 1 | 99,3 | 40 | 84,8 |
| | 2000 | 88 | 5 | 94,9 | 4 | 97,2 | 30 | 88,1 |
| DB 304 | 4000 | 80 | 0 | 100 | 1 | 99,2 | 1 | 99,6 |
| | 2000 | 88 | 7 | 92,7 | 7 | 95,2 | 4 | 98,4 |
| DB 312 | 4000 | 75 | 0 | 100 | 1 | 99,2 | 35 | 83,7 |
| | 2000 | 70 | 2 | 97,4 | 3 | 97,4 | 63 | 68,5 |
| DB 313 | 4000 | 74 | 0 | 100 | 0 | 100 | 7 | 96,7 |
| | 2000 | 104 | 0 | 100 | 0 | 100 | 8 | 97,3 |
| DB 314 | 4000 | 74 | 11 | 86,7 | 13 | 89,3 | 14 | 93,4 |
| | 2000 | 89 | 17 | 82,9 | 11 | 92,5 | 35 | 86,2 |
| DB 242 | 4000 | 105 | 0 | 100 | 0 | 100 | 1 | 99,7 |
| | 2000 | 111 | 1 | 99,2 | 2 | 98,9 | 5 | 99,1 |

TABLEAU 6 (suite)

| Numéro de code des composés | ppm i.a. | Moyenne de deux essais | | | | | | |
|---|---|---|---|---|---|---|---|---|
| | | Avant traitement | Jours après traitement | | | | | |
| | | | 2 | | 5 | | 10 | |
| | | Nombre d'acares | Nombre d'acares | %C | Nombre d'acares | %C | Nombre d'acares | %C |
| DB 261 | 4000 | 120 | 3 | 97,8 | 7 | 96,5 | 12 | 96,5 |
| | 2000 | 114 | 10 | 92,2 | 17 | 90,9 | 37 | 88,6 |
| DB 262 | 4000 | 123 | 1 | 99,3 | 8 | 96 | 11 | 96,9 |
| | 2000 | 110 | 8 | 93,5 | 22 | 87,8 | 33 | 89,5 |
| DB 263 | 4000 | 124 | 6 | 95,7 | 11 | 94,6 | 12 | 96,6 |
| | 2000 | 116 | 9 | 93,1 | 12 | 93,7 | 14 | 95,8 |
| DB 252 | 4000 | 118 | 1 | 99,2 | 0 | 100 | 32 | 90,5 |
| | 2000 | 94 | 2 | 98,1 | 2 | 98,7 | 49 | 81,7 |
| DB 254 | 4000 | 94 | 0 | 100 | 1 | 99,4 | 33 | 87,7 |
| | 2000 | 116 | 1 | 99,2 | 1 | 99,5 | 52 | 84,3 |
| DB 275 | 4000 | 97 | 27 | 75,1 | 26 | 83,7 | 39 | 85,9 |
| | 2000 | 70 | 32 | 59,1 | 34 | 70,5 | 61 | 69,5 |
| DB 285 | 4000 | 110 | 0 | 100 | 0 | 100 | 1 | 99,7 |
| | 2000 | 112 | 5 | 96 | 6 | 96,7 | 14 | 95,6 |
| DB 295 | 4000 | 92 | 1 | 99 | 0 | 100 | 10 | 96,2 |
| | 2000 | 110 | 3 | 97,6 | 3 | 98,3 | 29 | 90,8 |
| DB 305 | 4000 | 103 | 0 | 100 | 0 | 100 | 44 | 85 |
| | 2000 | 83 | 0 | 100 | 1 | 99,3 | 57 | 76 |
| DB 315 | 4000 | 100 | 3 | 97,3 | 7 | 95,7 | 21 | 92,6 |
| | 2000 | 87 | 17 | 82,5 | 18 | 87,4 | 45 | 81,9 |
| non traité | — | 76 | 85 | — | 125 | — | 217 | — |

Le tableau 6 montre clairement que la plupart des composés de formule I, III ou IV ont, de manière étonnante, une meilleure activité acaricide que le thiobenzoate de S-octyle qui est un composé acaricide connu.

Des essais complémentaires ont révélé que les composés DB 294, DB 242, DB 313, DB 252, DB 254 et DB 305 ont un meilleur effet destructeur ou une plus longue activité que le thiobenzoate de S-octyle. L'activité obtenue avec ces six composés était d'au moins 99%, 2 ou 10 jours après un traitement à une dose de 2000 ppm.

Un des composés les plus favorables suivant la présente invention est le composé DB 305 qui a encore accusé un degré de mortalité de 97% à une dose de 1000 ppm 10 jours après le traitement.

**Revendications**

1. Composition acaricide, contenant comme ingrédient actif, au moins un composé organique sulfuré répondant à la formule I suivante:

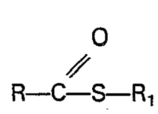

(I)

dans laquelle R et $R_1$ sont des radicaux organiques différents, caractérisée en ce que R désigne un radical

choisi parmi les radicaux méthyle, éthyle, propyle, butyle, hexyle et heptyle à chaîne droite ou ramifiée, le radical phényle éventuellement méthoxylé et le radical furyle-2 et $R_1$ désigne un radical choisi parmi les radicaux octyle, dodécyle et hexadécyle ou un groupe thioacylhexaméthylène de formule II suivante:

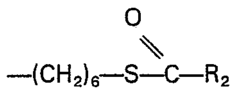

(II)

dans laquelle $R_2$ est identique à R, étant entendu que R ne représente pas un radical phényle, lorsque $R_1$ représente un radical octyle.

2. Composition acaricide suivant la revendication 1, caractérisée en ce qu'elle contient, comme ingrédient actif, au moins un S-thioester du type di(acylmercapto)-1,6-hexane de formule générale III suivante:

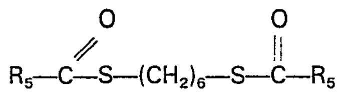

(III)

dans laquelle les symboles $R_5$ désignent des radicaux identiques choisis parmi les radicaux phényle et furyle-2.

3. Composition acaricide suivant la revendication 1, caractérisée en ce qu'elle contient, comme ingrédient actif, du thioisobutyrate de S-hexadécyle.

4. Composition acaricide suivant la revendication 1, caractérisée en ce qu'elle contient, comme ingrédient actif, du thiooctanoate de S-dodécyle.

5. Composition acaricide suivant la revendication 1, caractérisée en ce qu'elle contient, comme ingrédient actif, du di(pivaloylmercapto)-1,6-hexane.

6. Composition acaricide suivant la revendication 1 ou 2, caractérisée en ce qu'elle contient, comme ingrédient actif, du thiofuroate-2 de S-octyle.

**Patentansprüche**

1. Acaricides Mittel, enthaltend als Wirkstoff mindestens eine organische Schwefelverbindung entsprechend der folgenden Formel I

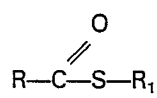

(I)

in der R und $R_1$ unterschiedliche organische Reste sind, dadurch gekennzeichnet, daß R ausgewählt ist aus Methyl, Ethyl, Propyl, Butyl, Hexyl und Heptyl, die geradoder verzweigtkettig sein können, dem gegebenenfalls methoxylierten Phenylrest und 2-Furyl und $R_1$ eine Gruppe bedeutet ausgewählt aus dem Octyl; Dodecyl- und Hexadecylrest oder eine Thioacyl-hexamethylengruppe der folgenden Formel II

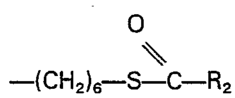

(II)

in der $R_2$ mit R gleich ist, mit der Maßgabe, daß R keinen Phenylrest bedeutet, wenn $R_1$ einen Octylrest bedeutet.

2. Acaricides Mittel nach Anspruch 1, dadurch gekennzeichnet, daß es als Wirkstoff mindestens einen S-Thioester der Art Di(acylmercapto)-1,6-hexan der allgemeinen Formel III

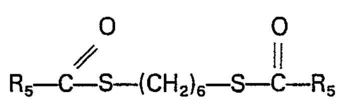

(III)

enthält, in der die Symbole $R_5$ gleiche Reste bedeuten, ausgewählt aus dem Phenyl-und dem 2-Furylrest.

3. Acaricides Mittel nach Anspruch 1, dadurch gekennzeichnet, daß es als Wirkstoff Thioisobuttersäure-S-hexadecylester enthält.

4. Acaricides Mittel nach Anspruch 1, dadurch gekennzeichnet, daß es als Wirkstoff Thiooctansäure-S-dodecylester enthält.

5. Acaricides Mittel nach Anspruch 1, dadurch gekennzeichnet, daß es als Wirkstoff Di(pivaloylmercapto)-1,6-hexan enthält.

6. Acaricides Mittel nach Anspruch 1 oder 2, dadurch gekennzeichnet, daß es als Wirkstoff 2-Thiofuransäure-S-octylester enthält.

**Claims**

1. Acaricidal composition containing, as active ingredient, at least a sulphur-containing organic compound of the following formula I:

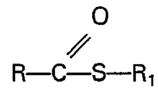

(I)

in which R and $R_1$ are different organic radicals, characterized in that R represents a radical selected among the methyl, ethyl, propyl, butyl, hexyl and heptyl radicals having a straight or branched chain, the possibly methoxylated phenyl radical and the furyl-2 radical, and $R_1$ represents a radical selected among the octyl, dodecyl and hexadecyl radicals or a thioacylhexamethylene group of the following formula II:

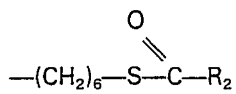

(II)

in which $R_2$ is identical to $R_1$, with the proviso that R does not represent a phenyl radical, when $R_1$ represents an octyl radical.

2. Acaricidal composition according to claim 1, characterized in that it contains, as active ingredient, at least a S-thioester of the di(acylmercapto)-1,6-hexane type of the following general formula III:

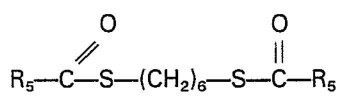

(III)

in which the $R_5$ symbols represent identical radicals selected among the phenyl and furyl-2 radicals.

3. Acaricidal composition according to claim 1, characterized in that it contains S-hexadecyl thioisobutyrate as active ingredient.

4. Acaricidal composition according to claim 1, characterized in that it contains S-dodecyl thiooctanoate as active ingredient.

5. Acaricidal composition according to claim 1, characterized in that it contains di(pivaloylmercapto)-1,6-hexane as active ingredient.

6. Acaricidal composition according to claim 1 or 2, characterized in that it contains S-octyl thiofuroate-2 as active ingredient.